# EUROPEAN PATENT SPECIFICATION

(11) **EP 0 805 647 B1**
(45) Date of publication and mention of the grant of the patent: **30.08.2000**
(21) Application number: 96900653.5
(22) Date of filing: 24.01.1996
(51) Int. Cl.: A61B 5/00, G01N 21/47

(54) **NON-INVASIVE MEDICAL SENSOR**
NICHTINVASIVER MEDIZINISCHER SENSOR
CAPTEUR MEDICAL NON INVASIF

(30) Priority: 27.01.1995 GB 9501663
(43) Date of publication of application: 12.11.1997
(73) Proprietor: Critikon Company, L.L.C., Tampa, Florida 33631 (US)
(72) Inventor: EVANS, Peter, Dilwyn, Cardiff CF3 7ET (GB); THOMAS, Gary, Anthony, Cardiff CF4 8EU (GB)
(74) Representative: Mercer, Christopher Paul
(86) International application number: GB9600140
(87) International publication number: WO9622728

(56) References cited:
- EP-A- 0 374 844
- EP-A- 0 442 011
- EP-A- 0 613 652
- WO-A-91/17697
- DE-U- 9 203 101

## Description

The present invention relates to apparatus for the non-invasive quantitative measurement of a substance in a human or animal body, and in particular to apparatus for such measurement utilising electromagnetic radiation.

The present invention is directed to an improvement of the system described in published international patent application WO 91/17697.

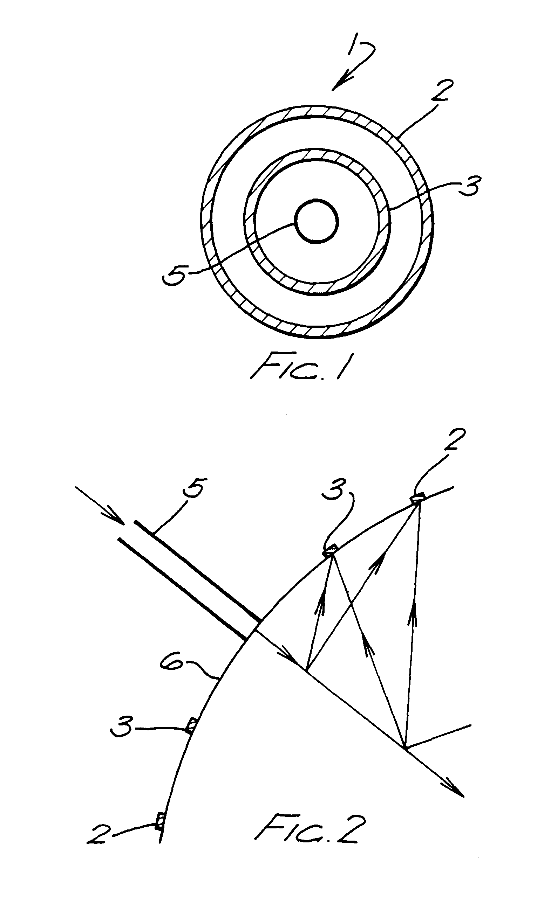

In that system, there is provided an arrangement as shown in Figures 1, 2 and 3 in which a sensor, generally designated 1, consists of a central emitter core 5 carrying light from four sub-emitter laser-diodes each capable of emitting infra-red radiation at a discrete wavelength within the range 700 to 1300nm. Arranged concentrically around the core 5 is a primary inner annular photodetector ring 3 and, spaced therefrom, a secondary outer annular photodetector ring 2.

The photodetector rings 2, 3 are connected to respective channels 7, 8 of suitable electronic amplification and signal processing circuitry. The processed output signals are then passed via signal steering circuitry 9 and an analog to digital converter 12 to a microprocessor control unit 13 programmed to calculate set algorithms dependent on the ratio of the two output signals, thus enabling a quantitative measurement of the amount of a substance in living tissue to be obtained. This measurement is then recorded or displayed on a VDU 10 and a chart recorder 11. Data can also be stored on a computer 16.

A laser power supply drive and firing circuitry 14 is provided, and this, together with the signal processing channels, 7, 8, is controlled by a timing sequen' controller 15 connected to the microprocessor control 13.

This system assumes the overall detection sensitivities of the two channels, i.e. the optical coupling efficiencies between the body and the two detectors and the gains of the two detector channels, to be precisely matched, or at least well defined. If matched, this allows the detection sensitivities between the two channels effectively to cancel each other out. If the detection sensitivities are well defined, this allows the final results to be corrected.

Thus, the signal attenuation measured between the photodetectors 2, 3 should be due to absorption occurring in the measurement region.

However, over the duration of a measurement, it is quite likely that moisture may build up under the sensors which may alter the coupling efficiencies of the two detectors independently.

It would therefore be desirable to provide an arrangement which attempts to overcome, or at least mitigate, this problem.

DE-U-9203101.3 describes a surface sensor arrangement for investigating biological and technical structures. Various emitter-sensor configurations are described. In one arrangement, six detectors are arranged on a first circle at the centre of which is disposed a first emitter, with further emitters being arranged on a second circle surrounding the first circle.

In accordance with the present invention there is provided apparatus for the non-invasive monitoring of a substance in living tissue as defined in Claim 1.

A preferred embodiment of the present invention will now be described with reference the accompanying drawings, in which:
Figure 1 is a schematic plan view of conventional apparatus;
Figure 2 is a cross-section through the apparatus of Figure 1 in use;
Figure 3 is a block diagram of a conventional control system for use with the apparatus shown in Figures 1 and 2;
Figure 4 is a cross-section through the apparatus of a preferred embodiment of the present invention; and
Figure 5 is a block diagram of a control system for use with the apparatus shown in Figure 4.

Referring to Figure 4, in which the same reference numerals are used as in Figures 1 and 2, an additional annular or part annular electromagnetic radiation emitter 17 is disposed between annular photodetectors 2 and 3 such that the spacing between the emitter 17 and the detectors 2, 3 is substantially equal.

Thus, when the photodetectors 2, 3 are caused to receive radiation solely from the second emitter 17, e.g. when the second emitter 17 is energised with the first emitter 5 not being energised, the output signals produced by the photodetectors 2, 3 provide a measure of the relative optical coupling and the relative gains of the two detector channels, and this information can be used to correct the output from the photodetectors 2, 3 occurring when the detectors 2, 3 are caused to receive radiation solely from the first emitter 5, e.g. when the first emitter 5 is energised with the second emitter 17 not being energised, such as occurs during an actual measurement process.

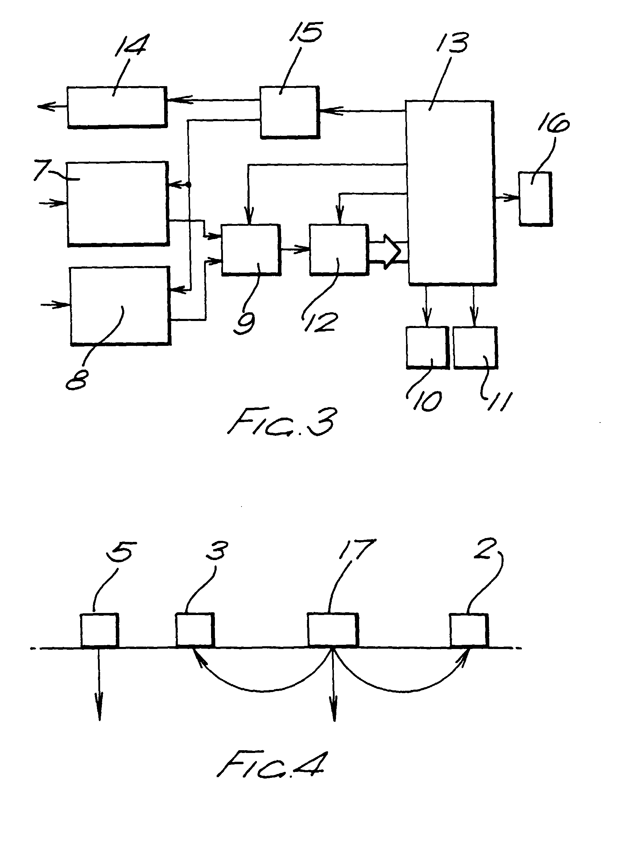

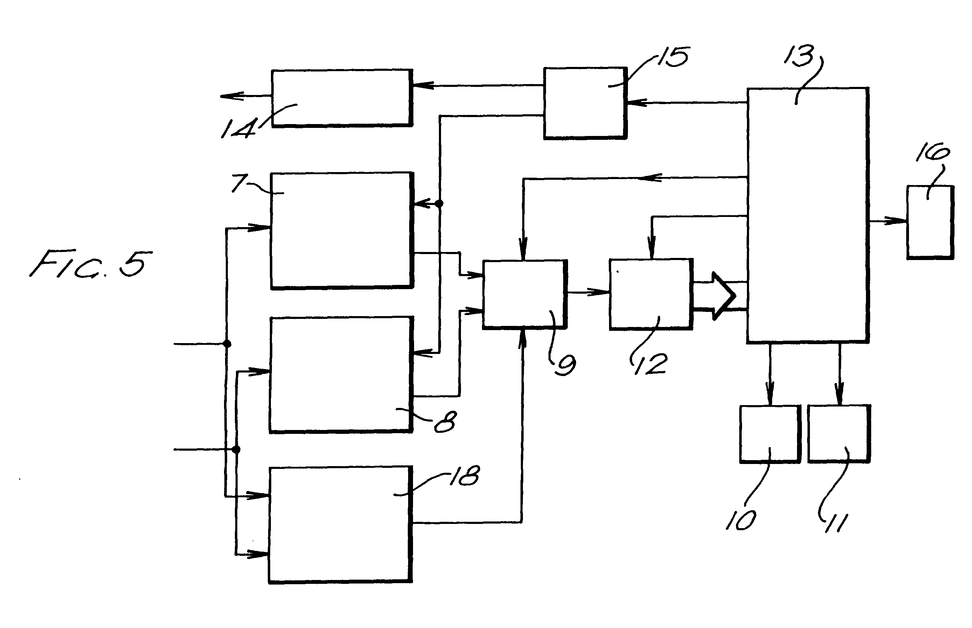

With reference to Figure 5, in which the same reference numerals are used as in Figure 3, the photodetectors 2, 3 are connected not only to respective channels 7, 8 of suitable electronic amplification and signal processing circuitry, but also to two inputs of processing circuitry 18, which processes the output signals occurring when the photodetectors 2, 3 are caused to receive radiation solely from the second emitter 17. This processing circuitry 18 generates an output which is fed to the signal steering circuitry 9 such that the processing of the output signals of the channels 7, 8 is made dependent on the output of the processing circuitry 18, such that the output signals are corrected for any differences in optical coupling or gains of the two detector channels.

Thus, any unequal gains which are present in the two detector channels, or any inequality in the optical couplings, are thereby compensated.

## Claims

1. Apparatus for the non-invasive monitoring of a substance in living tissue, the apparatus comprising:
a) first emitter means (5) capable of emitting electromagnetic radiation and being arrangeable in use in contact with skin, tissue or organ of a patient;
b) first radiation detection means (3) spaced from said first emitter means (5) and arrangeable in use in contact with the skin, tissue or organ of said patient;
c) means (8) for producing a first electrical output signal dependent on the intensity of the radiation detected by said first radiation detector means (3);
d) second radiation detection means (2) spaced from said first emitter means (5) by a distance greater than said spacing between said first radiation detection means (3) and said first emitter means (5) and arrangeable in use in contact with the skin, tissue or organ of said patient;
e) means (7) for producing a second electrical output signal dependent on the intensity of the radiation detected by said second radiation detection means (2); and
f) means (9) for processing said first and second output signals generated in response to radiation received from said first emitter means (5);
characterised in that said apparatus further comprises:
g) second emitter means (17) capable of emitting electromagnetic radiation and being arrangeable in use in contact with the skin, tissue or organ of said patient, and being spaced from said first (3) and second (2) radiation detection means by substantially the same distance;
said processing means (9) being arranged to process said first and second output signals in dependence on the values of said first and second output signals generated in response to radiation received from said second emitter means (17), thereby to provide a quantitative measurement of the amount of said substance in said living tissue which is compensated against differences in detection sensitivity between said first (3) and second (2) radiation detection means.

2. Apparatus as claimed in claim 1, wherein at least one of said first (3) and second (2) detection means is at least part annular.

3. Apparatus as claimed in claim 1 or claim 2, wherein said first (3) and second (2) detection means comprise annular detectors arranged concentrically around said first emitter means (5).

4. Apparatus as claimed in claim 2 or claim 3, wherein said second emitter means (17) is at least part annular.

5. Apparatus as claimed in any one of claims 2 to 4, wherein said second emitter means (17) comprises an annular emitter arranged concentrically around said first emitter means (5).

## Patentansprüche

1. Vorrichtung für die nicht-invasive Überwachung einer Substanz in lebendem Gewebe, wobei die Vorrichtung umfaßt:
a) erste Emitter- bzw. Sendemittel bzw. -einrichtungen (5), die fähig sind, elektromagnetische Strahlung auszusenden bzw. zu emittieren und die in Verwendung in Kontakt mit Haut, Gewebe oder einem Organ eines Patienten anordenbar sind;
b) erste Strahlungsdetektionsmittel bzw. -einrichtungen (3), die von den ersten Sendeeinrichtungen (5) beabstandet sind und die in Verwendung in Kontakt mit der Haut, Gewebe oder einem Organ des Patienten anordenbar sind;
c) Einrichtungen bzw. Mittel (8) zum Bilden eines ersten elektrischen Ausgabe- bzw. Ausgangssignals in Abhängigkeit von der Intensität der von den ersten Strahlungsdetektoreinrichtungen (3) detektierten Strahlung;
d) zweite Strahlungsdetektionsmittel bzw. - einrichtungen (2), die von den ersten Sendeeinrichtungen (5) um einen größeren Abstand beabstandet sind als der Abstand zwischen den ersten Strahlungsdetektionseinrichtungen (3) und den ersten Sendeeinrichtungen (5) und die in Verwendung in Kontakt mit der Haut, Gewebe oder einem Organ des Patienten anordenbar sind;
e) Einrichtungen bzw. Mittel (7) zum Bilden eines zweiten elektrischen Ausgabe- bzw. Ausgangssignals in Abhängigkeit von der Intensität der durch die zweiten Strahlungsdetektionseinrichtungen (2) detektierten Strahlung; und
f) Einrichtungen bzw. Mittel (9) zum Verarbeiten der ersten und zweiten Ausgabesignale, die in Antwort auf die von den ersten Sendeeinrichtungen (5) empfangene Strahlung generiert wurden, dadurch gekennzeichnet, daß die Vorrichtung weiters umfaßt:
g) zweite Emitter- bzw. Sendemittel bzw. -einrichtungen (17), die fähig sind, elektromagnetische Strahlung auszusenden bzw. zu emittieren und die in Verwendung in Kontakt mit der Haut, Gewebe oder einem Organ des Patienten anordenbar sind und die von den ersten (3) und zweiten (2) Strahlungsdetektionseinrichtungen um im wesentlichen denselben Abstand beabstandet sind;
welche Verarbeitungseinrichtungen (9) angeordnet bzw. ausgebildet sind, um die ersten und zweiten Ausgabesignale in Abhängigkeit von den Werten der ersten und zweiten Ausgabesignale, die in Antwort auf die Strahlung, die von den zweiten Sendeeinrichtungen (17) erhalten wurden, zu verarbeiten, um dadurch eine quantitative Messung der Menge der Substanz in dem lebenden Gewebe, welche gegen Unterschiede in der Detektionsempfindlichkeit zwischen den ersten (3) und zweiten (2) Strahlungsdetektionseinrichtungen kompensiert bzw. ausgeglichen bzw. korrigiert ist, zur Verfügung zu stellen.

2. Vorrichtung nach Anspruch 1, worin wenigstens eine der ersten (3) und zweiten (2) Detektionseinrichtungen zumindest teilweise ringförmig ist.

3. Vorrichtung nach Anspruch 1 oder 2, worin die ersten (3) und zweiten (2) Detektionseinrichtungen ringförmige Detektoren umfassen, die konzentrisch um die ersten Sendeeinrichtungen (5) angeordnet sind.

4. Vorrichtung nach Anspruch 2 oder 3, worin die zweiten Sendeeinrichtungen (17) wenigstens teilweise ringförmig sind.

5. Vorrichtung nach einem der Ansprüche 2 bis 4, worin die zweiten Sendeeinrichtungen (17) einen ringförmigen Sender umfassen, der konzentrisch um die ersten Sendeeinrichtungen (5) angeordnet ist.

## Revendications

1. Dispositif destiné à contrôler de manière non invasive une substance dans un tissu vivant, le dispositif comprenant:
a) un premier moyen formant émetteur (5) pouvant émettre un rayonnement électromagnétique et pouvant être agencé, lors de l'utilisation, en contact avec la peau, un tissu ou un organe d'un patient ;
b) un premier moyen de détection de rayonnement (3) séparé dudit premier moyen formant émetteur (5) et pouvant être agencé, lors de l'utilisation, en contact avec la peau, un tissu ou un organe dudit patient ;
c) un moyen (8) destiné à produire un premier signal électrique de sortie en fonction de l'intensité du rayonnement détecté par ledit premier moyen de détection de rayonnement (3) ;
d) un second moyen de détection de rayonnement (2) séparé dudit premier moyen formant émetteur (5) d'une distance supérieure audit espacement entre ledit premier moyen de détection de rayonnement (3) et ledit premier moyen formant émetteur (5) et pouvant être agencé, lors de l'utilisation, en contact avec la peau, un tissu ou un organe dudit patient ;
e) un moyen (7) destiné à produire un second signal électrique de sortie en fonction de l'intensité du rayonnement détecté par ledit second moyen de détection de rayonnement (2) ; et
f) un moyen (9) destiné à traiter lesdits premier et second signaux de sortie produits en réponse au rayonnement reçu à partir dudit premier formant émetteur (5) ;
caractérisé en ce que ledit dispositif comprend, en outre:
g) un second moyen formant émetteur (17) pouvant émettre un rayonnement électromagnétique et pouvant être agencé, lors de l'utilisation, en contact avec la peau, un tissu ou un organe dudit patient, et étant séparé dudit premier (3) et dudit second (2) moyen de détection de rayonnement d'une distance sensiblement égale ;
ledit moyen de traitement (9) étant agencé de manière à traiter lesdits premier et second signaux de sortie en fonction des valeurs desdits premier et second signaux de sortie produits en réponse au rayonnement reçu à partir dudit second moyen formant émetteur (17), délivrant ainsi une mesure quantitative de la quantité de ladite substance dans ledit tissu vivant qui est compensée en termes de différences de sensibilité de détection entre lesdits premier (3) et second (2) moyens de détection de rayonnement.

2. Dispositif selon la revendication 1, dans lequel au moins un desdits premier (3) et second (2) moyens de détection est au moins partiellement annulaire.

3. Dispositif selon la revendication 1 ou la revendication 2, dans lequel lesdits premier (3) et second (2) moyens de détection comprennent des détecteurs annulaires agencés de manière concentrique autour dudit premier moyen formant émetteur (5).

4. Dispositif selon la revendication 2 ou la revendication 3, dans lequel ledit second moyen formant émetteur (17) est au moins partiellement annulaire.

5. Dispositif selon l'une quelconque des revendications 2 à 4, dans lequel ledit second moyen formant émetteur (17) comprend un émetteur annulaire agencé de manière concentrique autour dudit premier moyen formant émetteur (5).
